Europäisches Patentamt

European Patent Office

Office européen des brevets

(11) Publication number: **0 227 829**
**B1**

(12) **EUROPEAN PATENT SPECIFICATION**

(45) Date of publication of patent specification: **31.10.90**

(51) Int. Cl.⁵: **C 07 D 307/62**

(21) Application number: **86905089.8**

(22) Date of filing: **06.08.86**

(86) International application number:
**PCT/US86/01620**

(87) International publication number:
**WO 87/00839 12.02.87 Gazette 87/04**

(54) **PRODUCTION OF L-ASCORBIC ACID FROM 2-KETO-L-GULONIC ACID.**

(30) Priority: **09.08.85 US 764262**

(43) Date of publication of application:
**08.07.87 Bulletin 87/28**

(45) Publication of the grant of the patent:
**31.10.90 Bulletin 90/44**

(84) Designated Contracting States:
**AT BE CH DE FR GB IT LI NL SE**

(56) References cited:
**EP-A-0 091 134**
**CH-A- 187 934**

**Chemical Abstracts, volume 79, no.9, 3
September 1979, (Columbus, Ohio, US), see
page 352, abstract no. 53169z, & JP A 7315931**

(73) Proprietor: **The Lubrizol Corporation
29400 Lakeland Boulevard
Wickliffe, Ohio 44092 (US)**

(72) Inventor: **YODICE, Richard
5485C Wildwood Court
Willoughby, OH 44094 (US)**

(74) Representative: **Vossius & Partner
Siebertstrasse 4 P.O. Box 86 07 67
D-8000 München 86 (DE)**

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

**Description**

This invention relates to the production of *L*-ascorbic acid from 2-keto-*L*-gulonic acid.

The most successful and common method of producing *L*-ascorbic acid is based on a multi-step synthesis from *d*-glucose going through sorbose and 2-keto-*L*-gulonic acid as described by Reichstein and Grussner, Helv. Chim. Acta., 17, 311—328 (1934). *L*-ascorbic acid is obtained by heating 2-keto-*L*-gulonic acid in water at 100°, or by esterifying 2-keto-*L*-gulonic acid (L-xylo-2-hexulosonic acid), which is in turn converted into *L*-ascorbic acid by treatment with sodium methoxide in methanol, followed hy acidification with hydrogen chloride gas.

A summary of the various techniques for producing *L*-ascorbic acid is found in Advances in Carbohydrate Chemistry and Biochemistry, Vol. 37, pp. 79—197 (1980). *L*-ascorbic acid, aside from its use as a vitamin (Vitamin C), has numerous other industrial and commercial uses.

A Japanese patent publication relating to the production of *L*-ascorbic acid 48 (1973) — 15931, based on application number 87,371, filed October 5, 1970, is reported in Chemical Abstracts, Vol. 79, 1973, p. 352 (53169Z). This patent publication discloses the preparation of *L*-ascorbic acid by treating diacetone-2-keto-*L*-gulonic acid hydrate, 2-keto-*L*-gulonic acid hydrate, or 2-keto-*L*-gulonic acid with concentrated hydrochloric acid in the presence of inactive solvents and surfactants. By way of example, this publication discloses heating 100 grams of diacetone-2-keto-*L*-gulonic acid, 300 ml of benzene, 0.3 grams of stearyl-propylenediamine dioleate, and 10 ml of concentrated hydrochloric acid for five hours at 65°C to give 58.7 grams of *L*-ascorbic acid (purity 97.5%).

According to EP—A—0 091 134 L-ascorbic acid is prepared from sodium 2-keto-L-gulonate by introducing gaseous hydrogen chloride in a molar ratio to the sodium-2-keto-L-gulonate of about 1.5—2.0 into a mixture composed of the sodium 2-keto-L-gulonate, ethanol and acetone in a ratio of 1:0.25—1.00: 0.5—2.5 by weight at a temparture of about 25—75°C until precipitation of sodium chloride ends, removing the precipitated sodium chloride before L-ascorbic acid begins to crystalize out, maintaining the filtrate or supernatant at a temperature of about 25—75°C for a period of 5—100 hours and cooling it to yield crystals of L-ascorbic acid as a final product. Overall yields of about 60—82% are obtained.

The technical problem underlying the present invention is to provide a single step method for converting 2-keto-L-gulonic acid to L-ascorbic acid under conditions, which substantially decreases the reaction time and substantially increases the total yield of L-ascorbic acid.

The present invention relates to a method for producing L-ascorbic acid by reacting 2-keto-L-gulonic acid with an acid, characterized in that

(a) forming a slurry of 2-keto-L-gulonic acid and a surfactant in an aromatic, aliphatic or halogenated aliphatic solvent or a mixture thereof, wherein the ratio of water to organic solvent is up to 1:20 (v/v), and

(b) reacting the slurry with anhydrous hydrogen chloride gas at a temperature from 40°C to 80°C for 2 to 5 hours.

This method preferably utilizes a hydrate of 2-keto-*l*-gulonic acid. Thus, the slurry contains water only sufficient for the hydration of 2-keto-L-gulonic acid. The one-step synthesis reaction of the present invention will proceed well with essentially no free water added when utilizing a hydrate of 2-keto-*L*-gulonic acid. However, the reaction of the inventon will proceed well with very small amounts of water present such as water present due to processing conditions. Increasing the amount of free water increases reaction time and/or decreases yield. This undesirable effect is due in part to the decomposition of the *L*-ascorbic acid which takes place in the presence of an aqeuous acid.

The 2-keto-*L*-gulonic acid intermediate can be obtained by a number of well known synthetic routes starting from commercially available *L*-sorbose. Protection of *L*-sorbose as a diacetonide can be accomplished in acetone using dimethoxypropane with a sulfuric acid catalyst. The diacetone-1-sorbose can then be oxidized using basic sodium hypochlorite in the presence of a nckel chloride catalyst to give diacetone-2-ketogulonic acid. Removal of the protecting group of the diacetone-2-ketogulonic acid can be accomplished by heating it in distilled water to produce 2-keto-*L*-gulonic acid.

A convenient method for introducing minimum amounts of water into the reaction is through the use of a hydrate of 2-keto-*l*-gulonic acid. While 2-keto-*L*-gulonic acid $1H_2O$ is a true hydrate, further water may be associated with the solid as unbound water. Drying 2-keto-*L*-gulonic acid in a vacuum at 50°C overnight gives 2-keto-*L*-gulonic acid $nH_2O$, where n is a hydrate from about 0 to about 0.5. Air-drying overnight at ambient temperature gives 2-keto-*L*-gulonic acid $nH_2O$, where n is a hydrate from about 1 to about 2. A preferred material is produced by drying overnight at ambient temperatures to yield a hydrate of 2-keto-*L*-gulonic acid $nH_2O$ wherein n is about 1.5. There appears to be a correlation between the hydration state of 2-keto-*L*-gulonic acid and its conversion to *L*-ascorbic acid. Generally, as the hydration state of 2-keto-*L*-gulonic acid decreases, there is a corresponding decrease in its conversion to *L*-ascorbic acid. It should be noted that the dependence on hydration state is observed under substantially anhydrous (i.e., water starved) conditions. Very small amounts of water can be added to the reaction medium to aid in cyclization; however, no additional water need be added if the hydration state is sufficiently high (e.g., at least about 1.0 and, preferably, about 1.5), providing other reaction criteria are met.

*L*-ascorbic acid is known to decompose in aqueous acids via a first order reaction with a half life of 5 hours at 70°C. Therefore, minimizing the time that *L*-ascorbic acid is in contact with aqueous hydrochloric acid is critical. This is accomplished in the present invention by slurrying the 2-keto-*L*-gulonic acid in a

2

supporting organic layer and using a minimum amount of water as a reaction medium. The supporting organic layer can be an aromatic solvent, an aliphatic solvent, a halogenated aliphatic solvent or a mixture thereof. Representative aromatic solvents include toluene, xylene, benzene, ethyl benzene and mixtures thereof. A preferred organic solvent is toluene, either alone or in combination with other organic or aliphatic solvents. Representative aliphatic solvents include hexane, heptane, octane, nonane, decane, dodecane and mixtures thereof. Representative halogenated aliphatic solvents are dichloromethane, chloroform, carbon tetrachloride and dichloroethane.

Nonionic, cationic and anionic surfactants can be used in this invention; however, nonionic and cationic surfactants are preferred. Examples of surfactants which can be used in the present invention include quaternary ammonium ions, phosphonium ions, amines (which are cationic sources), crown ethers, ethoxylated alcohols, cryptands, aminopolyethers, phosphorylsulfoxides, certain naturally occurring ionophores, glycerol esters, sorbitan esters and mixtures thereof. Representative surfactants include hexadecyltrimethylammonium chloride, oleylamine, tetrabutylammonium chloride, benzyltriethylammonium chloride, trioctylmethylammonium chloride, tricaprylmethylammonium chloride, tetrabutylammonium hydrogen sulfate and mixtures thereof. Typical amounts of surfactants are from about 1 mg to about 250 mg for every gram of 2-keto-L-gulonic acid.

Those skilled in the art will recognize other surfactant materials that are useful in connections with the reaction of the invention. A general listing of surfactants can be found in *McCutcheon's Emulsifier & Detergents*, 1983 McCutcheon Pub. Co.

The substantially anhydrous acid catalyst is hydrogen chloride gas. The advantages of anhydrous hydrogen chloride gas over other acids are cost, ease of removal from the L-ascorbic acid, low side product formation relative to other mineral acids, and shorter reaction times. In reactions where minute quantities of water are present, such as when the water of hydration of 2-keto-L-gulonic acid serves as the aqueous phase, only enough substantially anhydrous hydrogen chloride gas to saturate this water may be necessary to produce L-ascorbic acid in high yield.

The yield and the purity of the L-ascorbic acid are sensitive to the time and temperature of reaction. The reaction temperature is in the range of from 40°C to 80°C. The time of reaction is in the range of from 2 hours to 5 hours. In general, lower reaction temperatures give poorer conversions requiring longer reaction times. In general, higher temperatures give acceptable conversions but lower crude yields (decomposition may occur). A preferred reaction time is from about 2 hours to about 4 hours. A preferred reaction temperature is from about 60°C to about 70°C. A reaction time of about 3 hours at a temperature of about 65°C produces high crude yields of L-ascorbic acid. The reaction can, of course, be conducted at atmospheric or superatmospheric pressure.

Since the reaction of the invention proceeds best in substantially anhydrous conditions, it is carried out by including the 2-keto-L-gulonic acid (hydrate) in a slurry and bubbling hydrogen chloride gas through the slurry. Aqueous HCl is not used due to the decomposition of L-ascorbic acid it causes. However, small amounts of concentrated HCl can be used to facilitate the reaction. The materials used to form the slurry and thus aid in promoting the reactions of the invention may vary substantially depending on factors such as the end use the L-ascorbic acid will be put to, (e.g., industrial, pharmaceutical, etc.). Accordingly, different materials may be used depending on economic and purity requirements. Depending on the ultimate application of the L-ascorbic acid, it will, of course be necessary to use materials (e.g., solvents, surfactants, reaction intermediates, etc.) which comply with various state, federal or foreign regulations.

The invention is illustrated by the following examples.

## Example 1

Into a 25 ml, 3-neck flask is placed 20 ml of toluene and 50 mg of hexadecyltrimethylammonium chloride. The solution is heated to 65°C and 5.0 gms, 0.023 moles of 2-keto-L-gulonic acid (1.5 hydrate) is added all at once which forms a slurry. Hydrogen chloride gas is bubbled in at a rate of 80 ml/min for 3 hours. The toluene is then removed by rotary evaporation under reduced pressure at 30°C. The solid is diluted with 20 ml of toluene and again evaporated to ensure all water is removed. The solid is washed 2 times with toluene and dried under vacuum overnight. The yield is 4.18 gms, 0.0226 moles, 99%, of the tan, semi-hydrate of L-ascorbic acid. m.p. = 184—187°C; 93/0 wt% L-ascorbic acid/2-keto-L-gulonic (by high pressure liquid chromatography) $[\alpha]^{25} = +47.8°$ (c = 1 in methanol).

## Example 2

Into a 25 ml, 3-neck flask is placed 20 ml of toluene and 50 mg of hexadecyltrimethylammonium chloride. The solution is heated to 65°C and 5.0 gms of 2-keto-L-gulonic acid (1.5 hydrate) is added, followed immediately by 1.0 ml of concentrated HCl. Following this, HCl gas is blown through the slurry at a rate of 80 ml/minute for 2.5 hours. The toluene is removed at 30°C under reduced pressure. This step is repeated twice to ensure dryness. The light brown solid is dried in vacuum overnight. The yield is 4.32 gms., 0.0213 moles, 94%; m.p. = 180—184°C; 90/1 wt% L-ascorbic acid/2-keto-L-gulonic acid (by high pressure liquid chromatography).

## Example 3

Into a 25 ml, 3 neck flask is placed 20 ml of toluene, 1 ml of distilled water and 50 mg of hexadecyl-

EP 0 227 829 B1

trimethylammonium chloride. The mixture is heated to 65°C and 5.0 gms of 2-keto-*l*-gulonic acid (1.5 hydrate) is added all at once. With stirring, hydrogen chloride gas is bubbled through the solution for 5 hours. After cooling to 30°C toluene is removed by rotary evaporation at reduced pressure. After adding additional toluene and removing it two additional times, the tan solid is dried. The yield is 4.32 gms, 0.0213 moles, 94%, m.p. = 181—185°C; 85/1 wt% *L*-ascorbic acid/2-keto-*L*-gulonic acid (by high pressure liquid chromotography).

## Example 4

Into a 50 ml, 3-neck roundbottom flask is placed 20 ml of distilled water. The crude ascorbic acid, 2.0 gms is dissolved into the water at room temperature and 0.2 gms of decolorizing charcoal is added. The slurry is filtered through diatomaceous earth and the filter cake washed with 5 ml of water. The water is removed by rotary evaporation and the white solid is washed with toluene and dried in a vacuum oven. The yield is 1.90 gms of *L*-ascorbic acid; m.p. = 180—181°C.

**Claims**

1. A method for producing L-ascorbic acid by reacting 2-keto-L-gulonic acid with an acid, characterized in that

(a) forming a slurry of 2-keto-L-gulonic acid and a surfactant in an aromatic, aliphatic or halogenated aliphatic solvent or a mixture thereof, wherein the ratio of water to organic solvent is up to 1:20 (v/v), and

(b) reacting the slurry with anhydrous hydrogen chloride gas at a temperature from 40°C to 80°C for 2 to 5 hours.

2. A method according to Claim 1, wherein the slurry contains water only sufficient for the hydration of 2-keto-L-gulonic acid.

3. A method according to Claim 1, wherein the 2-keto-L-gulonic acid is used as a hydrate.

4. A method according to Claim 3, wherein the hydrate is 2-keto-L-gulonic acid·$nH_2O$ wherein n is from about 0.1 to about 2.0.

5. A method according to Claim 3, wherein the hydrate is 2-keto-L-gulonic acid·$nH_2O$ wherein n is from about 1.0 to about 1.75.

6. A method according to Claim 3, wherein the hydrate is 2-keto-L-gulonic acid·$nH_2O$ wherein n is about 1.5.

7. A method according to Claim 1, wherein the surfactant is selected from the group consisting of quaternary ammonium ions, phosphonium ions, amines, crown ethers, cryptands, aminophosphoryl-sulfoxides, ionophores, glycerol esters, ethoxylated alcohols, sorbitan esters and mixtures thereof.

8. A method according to Claim 7, wherein the surfactant is selected from the group consisting of quaternary ammonium halides, glycerol esters and mixtures thereof.

9. A method according to Claim 7, wherein the surfactant is selected from the group consisting of glycerol esters and mixtures thereof.

10. A method according to Claim 1, wherein the solvent is selected from the group consisting of toluene, xylene, hexane, heptane, octane, nonane, decane and mixtures thereof.

11. A method according to Claim 10, wherein the solvent is toluene.

12. A method according to Claim 1, wherein the slurry is reacted for 2 to 4 hours.

13. A method according to Claim 1, wherein the reaction is carried out at a superatmospheric pressure.

14. A method according to Claim 1, wherein the 2-keto-L-gulonic acid has a hydration of about 1.5, the aromatic solvent is toluene, and the surfactant is a sorbitan ester.

15. A method according to Claim 1, wherein the L-ascorbic acid obtained is purified.

**Patentansprüche**

1. Verfahren zur Herstellung von L-Ascorbinsäure durch Umsetzung von 2-Keto-L-gulonsäure mit einer Säure, dadurch gekennzeichnet, daß

(a) eine Aufschlämmung von 2-Keto-L-gulonsäure und einem Netzmittel in einer aromatischen aliphatischen oder halogenierten aliphatischen Lösungsmittel oder einem Gemisch davon hergestellt wird, wobei das Verhältnis von Wasser zu organischem Lösungsmittel bis zu 1:20 (Vol/Vol) beträgt, und

(b) die Aufschlämmung mit wasserfreiem Chlorwasserstoff-Gas bei einer Temperatur von 40 bis 80°C 2 bis 5 Stunden umgesetzt wird.

2. Verfahren nach Anspruch 1, wobei die Aufschlämmung Wasser nur in ausreichender Menge für die Hydratation der 2-Keto-L-gulonsäure enthält.

3. Verfahren nach Anspruch 1, wobei die 2-Keto-L-gulonsäure als Hydrat eingesetzt wird.

4. Verfahren nach Anspruch 3, wobei das Hydrat 2-Keto-L-gulonsäure·$nH_2O$ ist, wobei n etwa 0,1 bis etwa 2,0 ist.

5. Verfahren nach Anspruch 3, wobei das Hydrat 2-Keto-L-gulonsäure·$nH_2O$ ist, wobei n etwa 1,0 bis etwa 1,75 ist.

6. Verfahren nach Anspruch 3, wobei das Hydrat 2-Keto-L-gulonsäure·$nH_2O$ ist, wobei n etwa 1,5 ist.

7. Verfahren nach Anspruch 1, wobei das Netzmittel ausgewählt ist aus der Gruppe der quaternärem

4

Ammoniumionen, Phosphoniumionen, Amine, Kronenether, Cryptanden, Aminophosphorylsulfoxide, Ionophore, Glycerinester, ethoxylierte Alkohole, Sorbitanester und Gemischen davon.

8. Verfahren nach Anspruch 7, wobei das Netzmittel ausgewählt ist aus der Gruppe der quaternären Ammoniumhalogenide, Glycerinester und Gemischen davon.

9. Verfahren nach Anspruch 7, wobei das Netzmittel ausgewählt aus der Gruppe der Glycerinester und ihren Gemischen.

10. Verfahren nach Anspruch 1, wobei das Lösungsmittel ausgewählt ist aus der Gruppe Toluol, Xylol, Hexan, Heptan, Octan, Nonan, Decan und Gemischen davon.

11. Verfahren nach Anspruch 10, wobei das Lösungsmittel Toluol ist.

12. Verfahren nach Anspruch 1, wobei die Aufschlämmung 2 bis 4 Stunden umgesetzt wird.

13. Verfahren nach Anspruch 1, wobei die Umsetzung bei Überdruck durchgeführt wird.

14. Verfahren nach Anspruch 1, wobei die 2-Keto-L-gulonsäure eine Hydratation von etwa 1,5 aufweist, das aromatische Lösungsmittel Toluol und das Netzmittel ein Sorbitan-ester ist.

15. Verfahren nach Anspruch 1, wobei die erhaltene L-Ascorbinsäure gereinigt wird.

## Revendications

1. Procédé de production d'acide L-ascorbique par la réaction d'acide 2-céto-L-gulonique avec un acide, caractérisé en ce que

a) on forme une suspension d'acide 2-céto-L-gulonique et d'un agent tensioactif dans un solvant aromatique, aliphatique ou aliphatique halogéné ou un mélange de ceux-ci, le rapport de l'eau au solvant organique s'élevant à des valeurs jusqu'à 1:20 (v/v) et

b) on fait réagir ladite suspension avec du chlorure d'hydrogène anhydre gazeux à une température de 40°C à 80°C pendant 2 à 5 heures.

2. Procédé selon la revendication 1, caractérisé en ce que la suspension ne contient que l'eau suffisant juste à l'hydratation de l'acide 2-céto-L-gulonique.

3. Procédé selon la revendication 1, caractérisé en ce que l'on utilise l'acide 2-céto-L-gulonique sous forme d'hydrate.

4. Procédé selon la revendication 3, caractérisé en ce que l'hydrate est l'acide 2-céto-L-gulonique·$nH_2O$, où n varie d'environ 0,1 à environ 2,0.

5. Procédé selon la revendication 3, caractérisé en ce que l'hydrate est l'acide 2-céto-L-gulonique·$nH_2O$, ou n varie d'environ 1,0 à environ 1,75.

6. Procédé selon la revendication 3, caractérisé en ce que l'hydrate est l'acide 2-céto-L-gulonique·$nH_2O$, où n est environ 1,5.

7. Procédé selon la revendication 1, caractérisé en ce que l'on choisit l'agent tensioactif dans le groupe forme par des ions d'ammonium quaternaire, des ions phosphonium, des amines, des éthers couronne, des cryptands, des aminophosphorylsulfoxydes, des ionophores, des esters de glycérol, des alcools éthoxylés, des esters de sorbitan et des mélanges de ceux-ci.

8. Procédé selon la revendication 7, caractérisé en ce que l'on choisit l'agent tensioactif dans le groupe formé d'halogénures d'ammonium quaternaire, d'esters de glycérol et de mélanges de ceux-ci.

9. Procédé selon la revendication 7, caractérisé en ce que l'on choisit l'agent tensioactif dans le groupe formé par des esters de glycérol et des mélanges de ceux-ci.

10. Procédé selon la revendication 1, caractérisé en ce que l'on choisit le solvant dans le groupe formé du toluène, du xylène, de l'hexane, de l'heptane, de l'octane, du nonane, du décane et de mélanges de ceux-ci.

11. Procédé selon la revendication 10, caractérisé en ce que solvant est le toluène.

12. Procédé selon la revendication 1, caractérisé en ce qu'on fait réagir la suspension pendant 2 à 4 heures.

13. Procédé selon la revendication 1, caractérisé en ce que l'on réalise la réaction à une pression supérieure à la pression atmosphérique.

14. Procédé selon la revendication 1, caractérisé en ce que l'acide 2-céto-L-gulonique possède un état d'hydratation d'environ 1,5, le solvant aromatique est le toluène et l'agent tensioactif est un ester de sorbitan.

15. Procédé selon la revendication 1, caractérisé en ce que l'on purifie l'acide L-ascorbique obtenu.